# EUROPEAN PATENT SPECIFICATION

(11) **EP 2 682 099 B1**
(45) Date of publication and mention of the grant of the patent: **02.09.2020**
(21) Application number: 13175125.7
(22) Date of filing: 04.07.2013
(51) Int. Cl.: A61K 9/00, A61K 9/14, A61K 31/40, A61K 31/439, A61K 31/4704, A61K 31/33

(54) **Dry Powder Inhaler Compositions Comprising Long Acting Muscorinic Antagonists**
Trockenpulverinhalatorzusammensetzungen mit langwirkenden Muskorinantagonisten
Compositions d'inhalateur de poudre sèche comprenant des antagonistes muscariniques à action prolongée

(30) Priority: 05.07.2012 TR 201207842; 12.09.2012 TR 201210438; 07.01.2013 TR 201300194
(43) Date of publication of application: 08.01.2014
(73) Proprietor: Arven Ilac Sanayi Ve Ticaret A.S., Istanbul 34460 (TR)
(72) Inventor: Cifter, Ümit, 34460 Istanbul (TR); Türkyilmaz, Ali, 34460 Istanbul (TR); Mutlu, Onur, 34460 Istanbul (TR); Ramazanoglu, Gaye, 34460 Istanbul (TR)
(74) Representative: Mutlu, Aydin

(56) References cited:
- EP-A1- 1 894 568
- EP-A1- 1 944 018
- WO-A1-2012/050945
- WO-A2-2010/144628
- WO-A2-2011/048379
- WO-A2-2011/093819

## Description

### Field of the Invention

This invention relates to novel pharmaceutical compositions for inhalation comprising separately or together the long acting muscarinic antagonists (LAMAs) in the form of a dry powder in admixture with a pharmaceutically acceptable carrier and its use in the treatment of respiratory condition selected from asthma, chronic obstructive pulmonary disease (COPD) and other obstructive airways diseases. More particularly, the pharmaceutically acceptable carrier comprises a mixture of fine particles having a mean (d50) particle size of 1 to 10 µm and coarse particles having a mean (d50) particle size of 10 to 100 µm wherein the weight ratio of the fine particles is min. 20% by weight of the total amount of the carrier.

In addition, the present invention relates to novel pharmaceutical composition for inhalation based on combinations of long acting beta agonists, short acting beta-2 agonists, corticosteroids or a combination of two or more of them.

### Background of the Invention

The delivery of the active substances, such as long acting muscarinic antagonists (LAMAs), which may show high efficiency even at low doses to the lungs in efficient and sufficient amounts so as to obtain the desired effects is of great importance because it is considerably difficult to deliver sufficient amounts of these active substances including LAMA group and especially tiotropium, glycopyrronium, ipratropium, aclidinium, oxitropium or daratropium to the lungs as they are very small in amount per dose required for the treatment. Therefore, said active substances have to be diluted with pharmaceutically acceptable carriers.

Pharmaceutically acceptable carriers are used as a flow aid and facilitate the dose of the active substances into the lungs. Therefore the properties of the particles of the carrier play an important role in the formulation of dry powder inhaler (DPI). Thus, carriers should be carefully selected, designed and controlled for the use in a dry powder inhalation formulation.

Accordingly, formulations of dry powder Inhalers (DPI) must fulfill a set of requirements, whereby in particular the following are to be considered:

### Content uniformity of the active drug:

In a single dose system, each capsule or blister needs to contain the same amount of drug. In a multi dose system, the same amount of drug must be released every time it is administered, to guarantee that the patient receives the same dose each time. The presence of carrier, and its structure (i.e. fine & coarse particle sizes and their weight ratio to each other), promotes content uniformity in what is generally a low-dosage medication.

### flowability:

The design of the device, the characteristics of the active and the filling platform to be used will determine the appropriate characteristics of the carrier that will be needed. The flow properties of the formulation will be important to ensure that the overall device functions in the correct way and provides consistent performance. The choice of carrier is essential in ensuring that the device works correctly and delivers the right amount of active to the patient. Therefore to use a carrier in two different particle sizes (fine and coarse) is essential. Moreover, the percentage of the fine particles amoung total excipient is also essential.

### dose consistency:

DPI devices have to show consistent dose uniformity in order to guarantee that all doses from the device contain the correct quantity of the active. Regardless of a patient's inhalation ability, it is essential that the dose released by the DPI device is exactly the same every time. Therefore, using the carrier with the right properties in the formulation assists dose-consistent delivery.

To fulfill all these requirements the formulations of DPIs needs to be adapted in particular by a careful selection of the carriers used. In order to meet these requirements, the inhalable, fine or microfine particles of active compounds are mixed with carriers. By means of the mixing process, the particle size of the carrier can also be changed such that a certain proportion is inhalable. The particle size of the carrier employed depends on the requirements and specifications of the powder inhaler which is intended for the administration of the formulation. It is true for these mixtures that during all required processing, transport, storage and dosage operations no segregation must take place, i.e. the active compound particles must not detach from their carrier particles. During dispersion in the inhaler, induced by the respiratory flow of the patient, the active compound particles, however, must be detached as effectively as possible, i.e. as quantitatively as possible, in order to be inhaled.

Additionally, the formulation should be a homogeneous mixture where the drug particles adhere to the carrier. The adhesion should not be too strong as the drug will not be able to release from the carrier particle during inhalation. Furthermore, a low dose of powder should be filled into the device and the drug should always be released in the same way. One of the main important parameters for the formulation is the particle size of the carrier. Therefore, it is found that using the right ratio of the fine (small) and coarse (large) particles of the selected carrier in the present formulations of the invention are essential.

Thus, the fine particle ratio directly affects the dose consistency of the active substance in the dry powder formulation. The desired effect would be gained if the active substance is sufficiently transmitted to the lungs in dry powder formulations that have good dose consistency. The weight ratio of the fine particles amoung the total excipient thus has a great importance.

In prior art the amount of the fine particles which usually used is between 5 to 15 % by weight amoung total excipient but it may not always be sufficient to carry out the efficient and the sufficient dose of the active substances to the lungs. However, when the amount of the fine particle carrier is more than %20 other problems may arise such as tendency to agglomeration.

However, the present inventors found that agglomerates formation may also occur during the preparation of the composition, i.e. during the blending of the active ingredient fine particles with coarser excipient particles. The formation of agglomerates is particularly critical when a low-dosage strength active ingredients, such as LAMAs is used. In fact, the lower is the active ingredient weight of the formulation, the higher is the detrimental effect of the agglomerates on the uniformity of the active ingredient in the powder blend. The lack of homogeneity of the powder, due to the formation of agglomerates, involves the risk of an over or under dosage. So the agglomeration, together with other properties such as high adhesiveness degree, leads to problems in the manufacturing of a powder formulation provided with good dosage consistency when administered by DPIs.

In prior art there are some techniques which may prevent this agglomeration by preparing microparticles with a defined particle size that are obtained by pre-mixing or pre-milling. However, the preparation of said microparticles is a time-consuming step. Moreover the present inventors have found that such microparticles can face stability problems after storage of the final formulation.

There is still a need for carriers that are able to overcome the problems mentioned above and the problems related with interaction of carrier between the LAMAs and furthermore the problems related to pulmonary administration of drugs. It would be highly advantageous to provide formulations which can easily and homogenously disperse the active substances in the formulation giving rise to a good uniformity of distribution of the particles and hence an accurate dose consistency together with a good performance in terms of delivered dose and max. respirable fraction. This invention also proposes the possibility to obtain different compositions and composition of combinations for pulmonary administration having satisfactory properties in terms of increasing drug deposition or accelerating drug release rate in a safe and effective way.

The problem is solved by using the appropriate carrier which has the optimum weight ratio of the fine particles to coarse particles. This helps to control the flowability, the drug release from the device and helps to ensure the correct and consistent dosage of the active that reaches the lungs. Furthermore, the amount of the fine carrier particles has a great importance because this provides the higher efficiency of delivery of the active substances to lungs even if the inhalation pressure is lower (which depends on the patient).

### The Detailed Description of the Invention

This invention relates to novel pharmaceutical compositions for inhalation comprising separately or together, LAMAs in the form of dry powder in admixture with a pharmaceutically acceptable carrier which doesn't interact with the drugs. The present invention further relates to its use in the treatment of respiratory condition selected from asthma and chronic obstructive pulmonary disease (COPD) and other obstructive airways diseases.

The object of the present invention is to provide a dry powder formulation for inhalation which comprise LAMAs, allows highly efficient and sufficient amount of dosing when inhaled by the patients even in a low pressure. Therefore, the delivery of the inhalable active substance amount contained in the dry powder formulation is achieved with minimum possible variability in every inhalation.

WO 2011/093819 is focused on a pharmaceutical formulation comprising tiotropium, formoterol, ciclesodine and/or pharmaceutically acceptable derivatives. The main problem mentioned in WO 2011/093819 is low stability of the formulaiton, the solution proposed to overcome this problem is using tiotropium bromide having a water content less than or equal to 2,5%. According to the teaching of WO 2011/093819, the formulation comprising titotropium bromide with a water content less than or equal to 2,5%, formoterol fumarate, ciclesonide having a mean particle size in the range of 1,5 to 4,5 µm has desired flow properties.

Another object of the present invention is to prepare a dry powder formulation which comprise LAMAs, allows highly accurate dose consistency that provides the inhalable active substance to be in equal and accurate amounts in each blister or capsule during the manufacture process.

Accordingly, another object of the present invention is to provide dry powder compositions for inhalation which is stable throughout the shelf life, in other words, which prevents any chemical reaction between the active substance and carrier which may cause degradation of the active and furthermore resistant to humidity and extreme temperature which may occur during the manufacturing process.

Another main object of the present invention is to provide dry powder compositions for inhalation having an adequate content uniformity of the active substance comprising LAMAs in order to guarantee that the patient receives the same dose each time even in low-dosage formulations.

Another object of the present invention is to obtain the dose consistency of the active in order to guarantee that all doses from the device contain the correct quantity of the active. Using the carrier with the right properties and the right ratio in the formulation assists dose-consistent delivery. According to this preferred embodiment, the weight ratio of the fine carrier particles to coarse carrier particles, is between 0.01 - 0.60 by weight, preferably it is between 0.03 - 0.45 by weight, more preferably it is between 0.05 - 0.40 by weight, the more preferably it is between 0.10 - 0.35 by weight, most preferably it is 0.25 - 0.35 by weight.

According to this embodiment, pharmaceutically acceptable carrier comprise a mixture of fine particles having a mean (d₅₀) particle size of 1.0 to 10.0 µm and coarse particles having a mean (d₅₀) particle size of 10.0 to 100.0 µm wherein the amount of the fine particles is min. 20 % by weight of the total amount of the carrier. According to preferred embodiment the amount of the fine particles between 20 % to 30 % by weight of the total amount of the carrier, more preferably between 20 % to 25 % by weight of the total amount of the carrier. More specifically the amount of fine particles is min. or equal to 23% by weight of the total amount of the carrier.

As used here in, "particle size distribution" means the cumulative volume size distrubition as tested by any conventionally accepted method such as the laser diffraction method. "mean particle size", (d₅₀) means, the size at which 50% by volume of the particles and "d₉₀" means that the size at which %90 by volume of the particles and "d₁₀" means the size at which 10% by volume of the particles.

According to an embodiment of the invention, the fine particles of the said pharmaceutically acceptable carrier have a mean (d50) particle size of 1.0 to 7.0 µm and coarse particles have a mean (d50) particle size of 10.0 to 75.0 µm.

According to a further embodiment of the invention, more specifically the fine carrier particles of the said pharmaceutically acceptable carrier have a particle diameter of d₁₀ between 1.0 - 4.0 µm, d₅₀ between 4.0 - 7.0 µm and d₉₀ between 7.0 - 15.0 µm. The coarse carrier particles of the said pharmaceutically acceptable carrier have a particle diameter of d₁₀ between 10.0 - 40.0 µm, d₅₀ between 40.0 - 75.0 µm and d₉₀ between 75.0 - 200.0 µm.

The coarse carrier particles are used to prevent (re)agglomeration of the fine particles of active. To provide this effect, carrier with a particle size of approximately ten times that of the active is used. Generally, a monolayer of the active particles is formed on the larger carrier particles. Since the active and carrier will have to be separated during inhalation, the shape and the surface roughness of the carrier particles is of significant importance. Carrier particles with a smooth surface will separate from the active more easily than highly porous particles of equal size.

The fine carrier particles are used to help the active to reach the lungs in a safer way and higher doses. Because the surface energy is normally not equally spread over the carrier particle, the active will tend to concentrate on higher energy sites. This can make separation of the active from the carrier following pulmonary delivery more difficult, especially for low dose formulations. The presence of fine carrier paticles, smaller than 10.0 micron or 5.0 micron, will help to prevent this, as the high energy sites will be occupied by the fine carrier particles and the active will tend to attach to the low energy sites. It is found that lung deposition will increase with an increasing fraction of fine carrier particles. Accordingly a reduction in particle size (having finer particles) increases the fluidization energy and this enhances the increase of the amount of drug particles that will get into the lung.

The drug particles will then adhere to the lower adhesion sites and will be easier released during inhalation. With the addition of fines also the surface area increases significantly and the payload will be reduced. When the fine carrier particles are slightly coarser then the drug particles it could eliminate the friction forces between drug and carrier in the mixing process.

Another object of the present invention is to obtain good flowability of the formulations to ensure that the right amount of the active is delivered by the devices for DPIs. In other words, in order to guarantee consistent production of the formulations, mechanical filling of the powder inhaler, correct dosage and release by the powder inhaler the present invention provides free-flowing formulations by selecting the right carrier in the right particle sizes and the optimum weight ratio of the fine particles amoung the overall excipients/carriers.

Another object of the present invention is to prevent agglomeration by using appropriate carrier in an optimum weight ratio of fine particles to the total amount of the carrier. Active particles have fine or sometimes microfine particles which have a particle size less than 10 microns and often lower than 4 microns in order to penetrate into the deep lungs. These fine drug particles will also have a tendency to agglomerate. Although having fine particles is essential for deposition into the lower respiratory tract during inhalation, it is known that the finer are the particles, the stronger are the cohesion forces that increase the formation of agglomerates. For this reason powders for inhalation have been commonly formulated by mixing fine carrier particles with coarse carrier particles. However, to prevent these agglomeration problems present inventors find the optimum ratios even if the fine carrier particles are above 20 % amount the total carrier particles.

In a preferred embodiment according to the present invention, the pharmaceutically acceptable carrier is selected from the group comprising lactose, mannitol, spray dried mannitol, glucose, arabinose, trehalose, cellobiose, sorbitol, maltitol, xylitol, saccharose, maltose, dextrane or a combination of two or more of them. Preferably the carrier is lactose, glucose, mannitol or spray dried mannitol or mixtures thereof.

As a further embodiment, the carrier may be a mixture of lactose and mannitol, or a mixture of lactose and glucose or a mixture of mannitol and glucose or a mixture of lactose and trehalose, or a mixture of mannitol and trehalose, or a mixture of glucose and trehalose, or a mixture of lactose and sorbitol, or a mixture of mannitol and sorbitol, or a mixture of glucose and sorbitol, or a mixture of lactose and cellobiose, or a mixture of mannitol and cellobiose, or a mixture of glucose and cellobiose, or a mixture of lactose and maltitol, or a mixture of mannitol and maltitol, or a mixture of glucose and maltitol, or a mixture of lactose and arabinose, or a mixture of mannitol and arabinose, or a mixture of glucose and arabinose. In another embodiment of the invention mannitol can be spray dried mannitol in all above mixtures.

One of the advantages embodiments of this invention is directed to narrow particle size distribution of fine carrier particles which means the ratio between the mean particle size and d₉₀ is equal to or greater than 0,40. Preferably, the ratio between the median particle size and d₉₀ is between 0.45 and 0.50, more preferably it is 0.50 and 0.70. This ensures, assuming an appropriate diameter, a maximum deposition of the embedded drug in the trachea-bronchial and deep alveoli regions at normal inhalation rates.

Additionally, this narrow particle size distribution also applies to coarse carrier particles in the compositions of the present invention which is equal to or greater than 0.40. Preferably, the ratio of narrow particle size distribution is between 0.45 and 0.50, more preferably it is 0.50 and 0.70.

Bronchoconstriction and inflammation are also associated with bronchial plugging with secretions, which may be treated with long acting muscarinic antagonists (LAMAs). In a preferred embodiment LAMAs are selected from the group comprising tiotropium, glycopyrronium, ipratropium, aclidinium, oxitropium or a pharmaceutically acceptable salt or ester thereof, or in enantiomerically pure form or as a racemic mixture or a combination of two or more of them. Preferably the LAMAs can be tiotropium bromide, tiotropium chloride, tiotropium iodide, tiotropium methanesulphonate, tiotropium paratoluenesulphonate, tiotropium methyl sulphate, glycopyrronium bromide, glycopyrronium acetate, ipratropium bromide, aclidinium bromide, oxitropium bromide or a combination of two or more of them. More preferably the LAMA is tiotropium bromide.

According to a preferred embodiment of the present invention, the mean particle size (d₅₀) of LAMAs that described above is between 0.1 - 5.0 µm, preferably 1.0 - 3.0 µm.

To assist better patient compliance, combination products are still needed. It would be highly desirable, however, to provide a combination therapy suitable to reduce bronchial inflammation, bronchial constriction and bronchial secretions in a single product or dosage form. It would also be desirable to provide such a combination product or composition in a form whereby the correct dosage of the various components is easily and safely administered.

Asthma, chronic obstructive pulmonary disease and other related disorders have been known to be treated with beta-2 adrenergic receptor agonists as they provide a bronchodilator effect to the patients, resulting in relief from the symptoms of breathlessness. Beta-2 adrenergic receptor agonists can be short acting for immediate relief, or long acting for long-term prevention, of asthma symptoms. Long actings are long acting beta agonists (LABA) whose effect lasts for 12 hours or more. Therefore, in a preferred embodiment of the invention is to provide dry powder compositions comprising LAMAs in combination with LABAs which are selected from the group comprising salmeterol, formoterol, arformoterol, indacaterol, olodaterol, vilanterol, carmoterol, bambuterol or a pharmaceutically acceptable salt or ester thereof, or in enantiomerically pure form or as a racemic mixture or a combination of two or more of them. Preferably LABAs can be salmeterol xinofoate, arformoterol tartrate, indacaterol tartrate, olodaterol hydrochloride, vilanterol trifenatate, carmoterol hydrochloride, bambuterol hydrochloride, formoterol fumarate or a combination of two or more of them.

Short actings are the short acting beta-2 agonists (SABAs). They are bronchodilators. They relax the muscles lining the airways that carry air to the lungs within 5 minutes, increasing airflow and making it easier to breathe. They relieve asthma symptoms for 3 to 6 hours. They do not control the inflammation. Therefore, in a preferred embodiment of the invention is to provide dry powder compositions comprising LAMAs in combination with SABAs which are selected from the group comprising salbutamol, levosalbutamol, terbutaline, pirbuterol, procaterol, fenoterol, biltolterol, ritodrine, metaproterenol or a pharmaceutically acceptable salt or ester thereof, or in enantiomerically pure form or as a racemic mixture or a combination of two or more of them. Preferably SABAs can be salbutamol sulphate, salbutamol hemi-sulphate, levosalbutamol sulphate, terbutaline sulfate, pirbuterol hydrochloride, pirbuterol acetate, procaterol hydrochloride, fenoterol hydrobromide, bitolterol mesylate, ritodrine hydrochloride, metaproterenol sulfate or a combination of two or more of them.

Whilst it is also known that, beta-2 agonists provide symptomatic relief of bronchoconstriction in patients, another component of asthma, i. e. inflammation, often requires separate treatment. According to this, involves treatment with a steroid. Treatment with an inhaled corticosteroid is considered one of the most potent and effective therapies currently available for persistent asthma. Therefore, another preferred embodiment of the invention is to provide dry powder compositions comprising LAMAs in combination with inhaled corticosteroids which are selected from the group comprising fluticasone, ciclesonide, budesonide, mometasone, beclomethasone, triamcinolone, flunisolide, dexamethasone or a pharmaceutically acceptable salt or ester thereof, or in enantiomerically pure form or as a racemic mixture or a combination of two or more of them. Preferably, the corticosteroids can be fluticasone propionate, fluticasone furoate, ciclesonide, budesonide, mometasone furoate, beclomethasone dipropionate, triamcinolone acetonide, flunisolide acetate, dexamethasone sodium phosphate or a combination of two or more of them.

According to above preferred embodiments of the present invention, the said pharmaceutical compositions may further comprise one or more additional active agents selected from long acting beta agonists, short acting beta-2 agonists, inhaled corticosteroids or a combination of two or more of them.

Therefore, in a preferred embodiment of the invention, the pharmaceutical compositions comprise LAMAs and long acting beta agonists, or comprise LAMAs and short acting beta-2 agonists, or comprise LAMAs and inhaled corticosteroids which are given detailed above.

We have also found that certain therapeutic three-in-one combinations further comprising specific, LABAs, SABAs, and/or inhaled corticosteroids surprisingly provide an enhanced, synergistic, effect in terms of treatment of bronchoconstriction, inflammation and mucous secretions of airways. Also the three-in-one combination therapy as provided by the present invention is an extremely patient-friendly combination, which results in maximum patient compliance and better control of asthma and chronic obstructive pulmonary disease than the known combinations or single therapies.

It will also be appreciated from the above that the respective therapeutic agents of the combined preparations can be administered simultaneously, either in the same or different pharmaceutical formulations, or separately or sequentially. If there is separate or sequential administration, it will also be appreciated that the subsequently administered therapeutic agents should be administered to a patient within a time scale so as to achieve, or more particularly optimise, the above referred to advantageous synergistic therapeutic effect of a combined preparation as present in a pharmaceutical product according to the present invention.

Therefore, in a further embodiment, the pharmaceutical compositions of the invention comprise long acting muscarinic antagonists, long acting beta agonists and short acting beta-2 agonists, or comprise long acting muscarinic antagonists, long acting beta agonists and inhaled corticosteorids, or comprise long acting muscarinic antagonists, short acting beta-2 agonists and inhaled corticosteorids.

According to a preferred embodiment of the invention, the therapeutically effective amount of said pharmaceutical compositions are administered once a day or administered twice a day.

According to a preferred embodiment, the pharmaceutical compositions are used for in the treatment of respiratory conditions selected from asthma and chronic obstructive pulmonary disease and other obstructive airways diseases. In particular, the combinations of compounds of the present invention are useful in the treatment of respiratory diseases and conditions comprising, asthma, acute respiratory distress syndrome, chronic pulmonary inflammatory disease, bronchitis, chronic bronchitis, chronic obstructive pulmonary (airway) disease, and silicosis; or immune diseases and conditions comprising: allergic rhinitis and chronic sinusitis.

According to a further embodiment, the pharmaceutical compositions are suitable for administration separately, sequentially or together in effective amounts, together with a moisture tight and high barrier sealed blister or together with a capsule

In particular, the blister comprises aluminium to prevent ingress of moisture whereby the fine particle fraction (FPF) of the pharmaceutical composition dose is preserved. Furthermore, the blister is a high barrier sealed against moisture. Thus, the blister does not release any water to the dose and ingress of moisture from the exterior into the container is thereby prevented.

In a further preferred embodiment of the invention, the dry powder is in a capsule, which can be a pharmaceutically acceptable natural or synthetic polymer such as gelatine or hydroxypropyl methylcellulose.

In a preferred embodiment, the pharmaceutical compositions are suitable for administration separately, sequentially or together in effective amounts, together with an inhalation device. The device is preferably dry powder inhaler including the blister or the capsule described above.

In a further embodiment, the device in which the pharmaceutical composition is within the blister comprise at least one lock mechanism, enabling the device to remain locked in both positions in which the device is ready for inhalation and the lid is in the closed position, and further enabling the device to setup again automatically, when the lid is closed.

In a further embodiment, the invention relates to a pharmaceutical kit comprising the LAMAs and one or more additional active agents, in separate unit dosage forms, said forms being suitable for administration separately, sequentially or together in effective amounts, together with one or more inhalation devices for administration of LAMAs and one or more additional active agents which are LABAs, SABAs and/or inhaled corticosteroids as described in detail above.

In a further embodiment, the process for making the pharmaceutical compositions for inhalation of the present invention comprises the following steps;
To obtain a homogenous mixture first half of the coarse lactose particles are added to a glass container later on fine lactose particles are added and active ingredients are added to this mixture and blended in a turbula shaker. Then this mixture is elected. This election is not a milling, the aim of this election is to obtain a homogenous mixture. Then the rest of the coarse lactose particles are added to this elected mixture during blending. Final powder mixture is furthermore blended and then filled into blisters or capsules.

This invention is further defined by reference to the following examples. In the following examples the LAMAs has the ratio between the median particle size (d₅₀) and d₉₀ is about 0.50. Although these examples are not intended to limit the scope of the present invention, it should be considered in the light of the description detailed above.

### Example - 1:

**Table 1**

| **Ingredients** | **Amount % (w/w)** |
|---|---|
| tiotropium | 0.01 - 10.0 |
| Fine lactose | 20.0 - 25.0 |
| coarse lactose | 25.0 - 80.0 |

Particle size of the tiotropium (µm):

| | | |
|---|---|---|
| d₁₀ : 0.10 - 1.0 | d₅₀: 1.0 - 3.0 | d₉₀: 3.0 - 5.0 |

Particle size of the fine lactose (µm):

| | | |
|---|---|---|
| d₁₀ : 1.0 - 4.0 | d₅₀: 4.0 - 7.0 | d₉₀: 7.0 - 15.0 |

Particle size of the coarse lactose (µm):

| | | |
|---|---|---|
| d₁₀ : 10 - 40 | d₅₀: 40 - 75 | d₉₀: 75 - 200 |

### Example - 2:

**Table 2**

| **Ingredients** | **Amount mg** |
|---|---|
| tiotropium | 0.018 |

| | **Amount % (w/w)** |
|---|---|
| Fine lactose | 23.0 |
| coarse lactose | 77.0 |

Particle size of the tiotropium (µm):

| | | |
|---|---|---|
| d₁₀ : 0.10 - 1.0 | d₅₀: 1.0 - 3.0 | d₉₀: 3.0 - 5.0 |

Particle size of the fine lactose (µm):

| | | |
|---|---|---|
| d₁₀ : 1.0 - 4.0 | d₅₀: 4.0 - 7.0 | d₉₀: 7.0 - 15.0 |

Particle size of the coarse lactose (µm):

| | | |
|---|---|---|
| d₁₀ : 10 - 40 | d₅₀: 40 - 75 | d₉₀: 75 - 200 |

### Example - 3:

**Table 3**

| **Ingredients** | **Amount % (w/w)** |
|---|---|
| ipratropium | 0.01 - 10.0 |
| Fine lactose | 20.0 - 25.0 |
| coarse lactose | 25.0 - 80.0 |

Particle size of the ipratropium (µm):

| | | |
|---|---|---|
| d₁₀ : 0.10 - 1.0 | d₅₀: 1.0 - 3.0 | d₉₀: 3.0 - 5.0 |

Particle size of the fine lactose (µm):

| | | |
|---|---|---|
| d₁₀ : 1.0 - 4.0 | d₅₀: 4.0 - 7.0 | d₉₀: 7.0 - 15.0 |

Particle size of the coarse lactose (µm):

| | | |
|---|---|---|
| d₁₀ : 10 - 40 | d₅₀: 40 - 75 | d₉₀: 75 - 200 |

### Example - 4:

**Table 4**

| **Ingredients** | **Amount % (w/w)** |
|---|---|
| glycopyrronium | 0.01 - 10.0 |
| Fine lactose | 20.0 - 25.0 |
| coarse lactose | 25.0 - 80.0 |

Particle size of the glycopyrronium (µm):

| | | |
|---|---|---|
| d₁₀ : 0.10 - 1.0 | d₅₀: 1.0 - 3.0 | d₉₀: 3.0 - 5.0 |

Particle size of the fine lactose (µm):

| | | |
|---|---|---|
| d₁₀ : 1.0 - 4.0 | d₅₀: 4.0 - 7.0 | d₉₀: 7.0 - 15.0 |

Particle size of the coarse lactose (µm):

| | | |
|---|---|---|
| d₁₀ : 10 - 40 | d₅₀: 40 - 75 | d₉₀: 75 - 200 |

### Example - 5:

**Table 5**

| **Ingredients** | **Amount % (w/w)** |
|---|---|
| daratropium | 0.01 - 10.0 |
| Fine lactose | 20.0 - 25.0 |
| coarse lactose | 25.0 - 80.0 |

Particle size of the daratropium (µm):

| | | |
|---|---|---|
| d₁₀ : 0.10 - 1.0 | d₅₀: 1.0 - 3.0 | d₉₀: 3.0 - 5.0 |

Particle size of the fine lactose (µm):

| | | |
|---|---|---|
| d₁₀ : 1.0 - 4.0 | d₅₀: 4.0 - 7.0 | d₉₀: 7.0 - 15.0 |

Particle size of the coarse lactose (µm):

| | | |
|---|---|---|
| d₁₀ : 10 - 40 | d₅₀: 40 - 75 | d₉₀: 75 - 200 |

### Example - 6:

**Table 6**

| **Ingredients** | **Amount % (w/w)** |
|---|---|
| oxitropium | 0.01 - 10.0 |
| Fine lactose | 20.0 - 25.0 |
| coarse lactose | 25.0 - 80.0 |

Particle size of the oxitropium (µm):

| | | |
|---|---|---|
| d₁₀ : 0.10 - 1.0 | d₅₀: 1.0 - 3.0 | d₉₀: 3.0 - 5.0 |

Particle size of the fine lactose (µm):

| | | |
|---|---|---|
| d₁₀ : 1.0 - 4.0 | d₅₀: 4.0 - 7.0 | d₉₀: 7.0 - 15.0 |

Particle size of the coarse lactose (µm):

| | | |
|---|---|---|
| d₁₀ : 10 - 40 | d₅₀: 40 - 75 | d₉₀: 75 - 200 |

### Example - 7:

**Table 7**

| **Ingredients** | **Amount % (w/w)** |
|---|---|
| aclidinium | 0.01 - 10.0 |
| Fine lactose | 20.0 - 25.0 |
| coarse lactose | 25.0 - 80.0 |

Particle size of the aclidinum (µm):

| | | |
|---|---|---|
| d₁₀ : 0.10 - 1.0 | d₅₀: 1.0 - 3.0 | d₉₀: 3.0 - 5.0 |

Particle size of the fine lactose (µm):

| | | |
|---|---|---|
| d₁₀ : 1.0 - 4.0 | d₅₀: 4.0 - 7.0 | d₉₀: 7.0 - 15.0 |

Particle size of the coarse lactose (µm):

| | | |
|---|---|---|
| d₁₀ : 10 - 40 | d₅₀: 40 - 75 | d₉₀: 75 - 200 |

### Example - 8:

**Table 8**

| **Ingredients** | **Amount % (w/w)** |
|---|---|
| LAMAs | 0.01 - 10.0 |
| LABAs | 0.01 - 10.0 |
| fine lactose | 20.0 - 25.0 |
| coarse lactose | 25.0 - 80.0 |

### Particle size of ;

| | | | |
|---|---|---|---|
| LAMAs (µm) | : d₁₀ : 0.10 - 1.0 | d₅₀: 1.0 - 3.0 | d₉₀: 3.0 - 5.0 |
| LABAs (µm) | : d₁₀ : 0.10 - 1.0 | d₅₀: 1.0 - 3.0 | d₉₀: 3.0 - 5.0 |
| fine lactosel (µm) | : d₁₀ : 1.0 - 4.0 | d₅₀: 4.0 - 7.0 | d₉₀: 7.0 - 15.0 |
| coarse lactose (µm) | : d₁₀ : 10 - 40 | d₅₀: 40 - 75 | d₉₀: 75 - 200 |

### Examples - 9:

**Table 9**

| **Ingredients** | **Amount % (w/w)** |
|---|---|
| LAMAs | 0.01 - 10.0 |
| SABAs | 0.01 - 10.0 |
| Fine lactose | 20.0 - 25.0 |
| coarse lactose | 25.0 - 80.0 |

### Particle size of;

| | | | |
|---|---|---|---|
| LAMAs (µm) | : d₁₀ : 0.10 - 1.0 | d₅₀: 1.0 - 3.0 | d₉₀: 3.0 - 5.0 |
| SABAs (µm) | : d₁₀ : 0.10 - 1.0 | d₅₀: 1.0 - 3.0 | d₉₀: 3.0 - 5.0 |
| fine lactosel (µm) | : d₁₀ : 1.0 - 4.0 | d₅₀: 4.0 - 7.0 | d₉₀: 7.0 - 15.0 |
| coarse lactose (µm) | : d₁₀ : 10 - 40 | d₅₀: 40 - 75 | d₉₀: 75 - 200 |

### Examples - 10:

**Table 10**

| **Ingredients** | **Amount % (w/w)** |
|---|---|
| LAMAs | 0.01 - 10.0 |
| Corticosteroids | 0.01 - 10.0 |
| Fine lactose | 20.0 - 25.0 |
| coarse lactose | 25.0 - 80.0 |

### Particle size of;

| | | | |
|---|---|---|---|
| LAMAs (µm) | : d₁₀ : 0.10 - 1.0 | d₅₀: 1.0 - 3.0 | d₉₀: 3.0 - 5.0 |
| Corticosteroids (µm) | : d₁₀ : 0.10 - 1.0 | d₅₀: 1.0 - 3.0 | d₉₀: 3.0 - 5.0 |
| fine lactosel (µm) | : d₁₀ : 1.0 - 4.0 | d₅₀: 4.0 - 7.0 | d₉₀: 7.0 - 15.0 |
| coarse lactose (µm) | : d₁₀ : 10 - 40 | d₅₀: 40 - 75 | d₉₀: 75 - 200 |

### Examples - 11:

**Table 11**

| **Ingredients** | **Amount % (w/w)** |
|---|---|
| LAMAs | 0.01 - 10.0 |
| LABAs | 0.01 - 10.0 |
| SABAs | 0.01 - 10.0 |
| Fine lactose | 20.0 - 25.0 |
| coarse lactose | 25.0 - 80.0 |

### Particle size of;

| | | | |
|---|---|---|---|
| LAMAs (µm) | : d₁₀ : 0.1 - 1.0 | d₅₀: 1.0 - 3.0 | d₉₀: 3.0 - 5.0 |
| LABAs (µm) | : d₁₀ : 0.10 - 1.0 | d₅₀: 1.0 - 3.0 | d₉₀: 3.0 - 5.0 |
| SABAs (µm) | : d₁₀ : 0.10 - 1.0 | d₅₀: 1.0 - 3.0 | d₉₀: 3.0 - 5.0 |
| fine lactose (µm) | : d₁₀ : 1.0 - 4.0 | d₅₀: 4.0 - 7.0 | d₉₀: 7.0 - 15.0 |
| coarse lactose (µm) | : d₁₀ : 10 - 40 | d₅₀: 40 - 75 | d₉₀: 75 - 200 |

### Examples -12:

**Table 12**

| **Ingredients** | **Amount % (w/w)** |
|---|---|
| LAMAs | 0.01 - 10.0 |
| LABAs | 0.01 - 10.0 |
| Corticosteroids | 0.01 - 10.0 |
| Fine lactose | 20.0 - 25.0 |
| coarse lactose | 25.0 - 80.0 |

### Particle size of;

| | | | |
|---|---|---|---|
| LAMAs (µm) | : d₁₀ : 0.1 - 1.0 | d₅₀: 1.0 - 3.0 | d₉₀: 3.0- 5.0 |
| LABAs (µm) | : d₁₀ : 0.10 - 1.0 | d₅₀: 1.0 - 3.0 | d₉₀: 3.0 - 5.0 |
| Corticosteroids (µm) | : d₁₀ : 0.10 - 1.0 | d₅₀: 1.0 - 3.0 | d₉₀: 3.0- 5.0 |
| fine lactose (µm) | : d₁₀ : 1.0 - 4.0 | d₅₀: 4.0 - 7.0 | d₉₀: 7.0 - 15.0 |
| coarse lactose (µm) | : d₁₀ : 10 - 40 | d₅₀: 40 - 75 | d₉₀: 75 - 200 |

### Examples -13:

**Table 13**

| **Ingredients** | **Amount % (w/w)** |
|---|---|
| LAMAs | 0.01 - 10.0 |
| SABAs | 0.01 - 10.0 |
| Corticosteroids | 0.01 - 10.0 |
| Fine lactose | 20.0 - 25.0 |
| coarse lactose | 25.0 - 80.0 |

### Particle size of;

| | | | |
|---|---|---|---|
| LAMAs (µm) | : d₁₀ : 0.1 - 1.0 | d₅₀: 1.0 - 3.0 | d₉₀: 3.0 - 5.0 |
| SABAs (µm) | : d₁₀ : 0.10 - 1.0 | d₅₀: 1.0 - 3.0 | d₉₀: 3.0- 5.0 |
| Corticosteroids (µm) | : d₁₀ : 0.10 - 1.0 | d₅₀: 1.0 - 3.0 | d₉₀: 3.0- 5.0 |
| fine lactose (µm) | : d₁₀ : 1.0 - 4.0 | d₅₀: 4.0 - 7.0 | d₉₀: 7.0 - 15.0 |
| coarse lactose (µm) | : d₁₀ : 10 - 40 | d₅₀: 40 - 75 | d₉₀: 75 - 200 |

## Claims

1. A pharmaceutical composition for inhalation comprising separately or together long acting muscarinic antagonists (LAMAs) in the form of a dry powder in admixture with a pharmaceutically acceptable carrier which comprise a mixture of fine particles having a mean (d50) particle size of 1.0 to 10.0 µm and coarse particles having a mean (d50) particle size of 10.0 to 100.0 µm wherein the amount of the fine particles is between 20% to 30% by weight of the total amount of the carrier.

2. The pharmaceutical composition according to claim 1, wherein the weight ratio of the fine particles to coarse particles is 0.25 - 0.35 by weight.

3. The pharmaceutical composition according to claim 1, wherein the fine particles of the said pharmaceutically acceptable carrier have a mean (d50) particle size of 1.0 to 7.0 µm and coarse particles have a mean (d50) particle size of 10.0 to 75.0 µm.

4. The pharmaceutical composition according to any of the preceding claims, wherein the pharmaceutically acceptable carrier is selected from the group comprising lactose, mannitol, spray dried mannitol, glucose, arabinose, trehalose, cellobiose, sorbitol, maltitol, xylitol, saccharose, maltose, dextran or a combination of two or more of them.

5. The pharmaceutical composition according to claim 4, wherein the pharmaceutically acceptable carrier is preferably lactose or glucose or mannitol or spray dried mannitol or mixtures thereof.

6. The pharmaceutical composition according to claim 4, wherein the pharmaceutically acceptable carrier is preferably mixture of lactose and mannitol or mixture of lactose and glucose or mixture of mannitol and glucose.

7. The pharmaceutical composition according to claim 1 and any of the preceding claims, wherein the mean particle size (d₅₀) of the LAMAs is between 0.10 - 5.0 µm.

8. The pharmaceutical composition according to claim 1 and any of the preceding claims, wherein the LAMAs are selected from the group comprising tiotropium, glycopyrronium, ipratropium, aclidinium, oxitropium, daratropium or a pharmaceutically acceptable salt or ester thereof, or in enantiomerically pure form or as a racemic mixture or a combination of two or more of them.

9. The pharmaceutical composition according to claim 8, wherein the LAMA is tiotropium or a pharmaceutically acceptable salt or ester thereof, or in enantiomerically pure form or as a racemic mixture.

10. The pharmaceutical composition according to claim 9, wherein the tiotropium is present in the form of the chloride, bromide, iodide, methanesulphonate, paratoluenesulphonate or methyl sulphate thereof, preferably in the form of bromide.

11. The pharmaceutical composition according to claim 1, further comprising one or more additional active agents selected from long acting beta agonists, short acting beta-2 agonists, corticosteroids or a combination of two or more of them.

12. The pharmaceutical composition according to claim 11, wherein the long acting beta agonists are selected from the group comprising salmeterol, formoterol, arformoterol, indacaterol, olodaterol, vilanterol, carmoterol, bambuterol or a pharmaceutically acceptable salt or ester thereof, or in enantiomerically pure form or as a racemic mixture or a combination of two or more of them.

13. The pharmaceutical composition according to claim 11, wherein the short acting beta-2 agonists are selected from the group comprising salbutamol, levosalbutamol, terbutaline, pirbuterol, procaterol, fenoterol, bitolterol, ritodrine, metaproterenol or a pharmaceutically acceptable salt or ester thereof, or in enantiomerically pure form or as a racemic mixture or a combination of two or more of them.

14. The pharmaceutical composition according to claim 11, wherein the corticosteroids are selected from the group comprising fluticasone, ciclesonide, budesonide, mometasone, beclomethasone, triamcinolone, flunisolide, dexamethasone or a pharmaceutically acceptable salt or ester thereof, or in enantiomerically pure form or as a racemic mixture or a combination of two or more of them.

15. The pharmaceutical composition according to any of the preceding claims, wherein the therapeutically effective amount of said pharmaceutical composition is administered once a day or twice a day.

16. The pharmaceutical composition according to any of the preceding claims, for use in the treatment of respiratory condition selected from asthma and chronic obstructive pulmonary disease and other obstructive airways diseases.

17. The pharmaceutical composition according to any of the preceding claims, wherein said pharmaceutical composition being suitable for administration separately, sequentially or together in effective amounts, together with a moisture tight and high barrier sealed blister.

18. The pharmaceutical composition according to any of the preceding claims, wherein said pharmaceutical composition being suitable for administration separately, sequentially or together in effective amounts, together with a capsule.

19. The pharmaceutical composition according to claim 17 or 18, wherein said pharmaceutical composition being suitable for administration separately, sequentially or together in effective amounts, together with a dry powder inhalation device.

20. The pharmaceutical composition according to claim 17, wherein said pharmaceutical composition being suitable for administration separately, sequentially or together in effective amounts, together with an inhalation device **characterized by** said device comprise at least one lock mechanism, enabling the device to remain locked in both positions in which the device is ready for inhalation and the lid is in the closed position, and further enabling the device to setup again automatically, when the lid is closed.

21. A pharmaceutical kit comprising the LAMAs and one or more additional active agents as defined in claim 11, in separate unit dosage forms, said forms being suitable for administration separately, sequentially or together in effective amounts, together with one or more inhalation devices for administration of LAMAs and one or more additional active agents.

## Patentansprüche

1. Pharmazeutische Zusammensetzung zur Inhalation, die separat oder zusammen langwirksame muskarinische Antagonisten (LAMAs) in Form eines Trockenpulvers im Gemisch mit einem pharmazeutisch akzeptablen Träger, der ein Gemisch von feinen Partikeln, die eine mittlere (d₅₀) Partikelgröße von 1,0 bis 10,0 µm aufweisen, und groben Partikeln, die eine mittlere (d₅₀) Partikelgröße von 10,0 bis 100,0 µm aufweisen, umfasst, wobei die Menge der feinen Partikel zwischen 20 bis 30 Gew.-% der Gesamtmenge des Trägers beträgt, umfasst.

2. Pharmazeutische Zusammensetzung gemäß Anspruch 1, wobei das Gewichtsverhältnis der feinen Partikel zu den groben Partikeln 0,25 bis 0,35, bezogen auf das Gewicht, beträgt.

3. Pharmazeutische Zusammensetzung gemäß Anspruch 1, wobei die feinen Partikel des pharmazeutisch akzeptablen Trägers eine mittlere (d₅₀) Partikelgröße von 1,0 bis 7,0 µm aufweisen und die groben Partikel eine mittlere (d₅₀) Partikelgröße von 10,0 bis 75,0 µm aufweisen.

4. Pharmazeutische Zusammensetzung gemäß einem der vorhergehenden Ansprüche, wobei der pharmazeutisch akzeptable Träger aus der Gruppe ausgewählt ist, die Laktose, Mannit, sprühgetrocknetes Mannit, Glucose, Arabinose, Trehalose, Cellobiose, Sorbit, Maltit, Xylit, Saccharose, Maltose, Dextran oder eine Kombination von zwei oder mehr von diesen umfasst.

5. Pharmazeutische Zusammensetzung gemäß Anspruch 4, wobei der pharmazeutisch akzeptable Träger vorzugsweise Laktose oder Glukose oder Mannit oder sprühgetrocknetes Mannit oder ein Gemisch hiervon ist.

6. Pharmazeutische Zusammensetzung gemäß Anspruch 4, wobei der pharmazeutisch akzeptable Träger vorzugsweise ein Gemisch von Laktose und Mannit oder ein Gemisch von Laktose und Glukose oder ein Gemisch von Mannit und Glukose ist.

7. Pharmazeutische Zusammensetzung gemäß Anspruch 1 und einem der vorhergehenden Ansprüche, wobei die mittlere Partikelgröße (d₅₀) der LAMAs zwischen 0,10 bis 5,0 µm beträgt.

8. Pharmazeutische Zusammensetzung gemäß Anspruch 1 und einem der vorhergehenden Ansprüche, wobei die LAMAs aus der Gruppe ausgewählt sind, die Tiotropium, Glycopyrronium, Ipratropium, Aclidinium, Oxitropium, Daratropium oder ein pharmazeutisch akzeptables Salz oder einen pharmazeutisch akzeptablen Ester hiervon oder in enantiomerenreiner Form oder als racemisches Gemisch oder eine Kombination von zwei oder mehr von diesen umfasst.

9. Pharmazeutische Zusammensetzung gemäß Anspruch 8, wobei das LAMA Tiotropium ist oder ein pharmazeutisch akzeptables Salz oder ein pharmazeutisch akzeptabler Ester hiervon oder in enantiomerenreiner Form oder als racemisches Gemisch.

10. Pharmazeutische Zusammensetzung gemäß Anspruch 9, wobei das Tiotropium in Form des Chlorids, Bromids, Iodids, Methansulfonats, para-Toluolsulfonats oder Methylsulfats, vorzugsweise in Form des Bromids, vorhanden ist.

11. Pharmazeutische Zusammensetzung gemäß Anspruch 1, die des Weiteren einen oder mehrere zusätzliche Wirkstoffe umfasst, die aus langwirksamen beta-Agonisten, kurzwirksamen beta-2-Agonisten, Corticosteroiden oder einer Kombination von zwei oder mehr von diesen ausgewählt sind.

12. Pharmazeutische Zusammensetzung gemäß Anspruch 11, wobei die langwirksamen beta-Agonisten aus der Gruppe ausgewählt sind, die Salmeterol, Formoterol, Arformoterol, Indacaterol, Olodaterol, Vilanterol, Carmoterol, Bambuterol oder ein pharmazeutisch akzeptables Salz oder einen pharmazeutisch akzeptablen Ester hiervon oder in enantiomerenreiner Form oder in Form eines racemischen Gemischs oder eine Kombination von zwei oder mehr von diesen umfasst.

13. Pharmazeutische Zusammensetzung gemäß Anspruch 11, wobei die kurzwirksamen beta-2-Agonisten aus der Gruppe ausgewählt sind, die Salbutamol, Levosalbutamol, Terbutalin, Pirbuterol, Procaterol, Fenoterol, Bitolterol, Ritodrin, Metaproterenol oder ein pharmazeutisch akzeptables Salz oder einen pharmazeutisch akzeptablen Ester hiervon, oder in enantiomerenreiner Form oder in Form eines racemischen Gemischs oder eine Kombination von zwei oder mehr von diesen umfasst.

14. Pharmazeutische Zusammensetzung gemäß Anspruch 11, wobei die Corticosteroide aus der Gruppe ausgewählt sind, die Fluticason, Ciclesonid, Budesonid, Mometason, Beclomethason, Triamcinolon, Flunisolid, Dexamethason oder ein pharmazeutisch akzeptables Salz oder einen pharmazeutisch akzeptablen Ester hiervon, oder in enantiomerenreiner Form oder in Form eines racemischen Gemischs oder eine Kombination von zwei oder mehr von diesen umfasst.

15. Pharmazeutische Zusammensetzung gemäß einem der vorhergehenden Ansprüche, wobei die therapeutisch wirksame Menge der pharmazeutischen Zusammensetzung einmal pro Tag oder zweimal pro Tag verabreicht wird.

16. Pharmazeutische Zusammensetzung gemäß einem der vorhergehenden Ansprüche zur Verwendung bei der Behandlung eines respiratorischen Zustands, der aus Asthma und chronischer obstruktiver Lungenerkrankung und anderen obstruktiven Atemwegserkrankungen ausgewählt ist.

17. Pharmazeutische Zusammensetzung gemäß einem der vorhergehenden Ansprüche, wobei die pharmazeutische Zusammensetzung für eine separate, sequenzielle oder gemeinsame Verabreichung in wirksamen Mengen, zusammen mit einem feuchtigkeitsdichten und eine hohe Barriere bildenden versiegelten Blister geeignet ist.

18. Pharmazeutische Zusammensetzung gemäß einem der vorhergehenden Ansprüche, wobei die pharmazeutische Zusammensetzung für eine separate, sequenzielle oder gemeinsame Verabreichung in wirksamen Mengen, zusammen mit einer Kapsel geeignet ist.

19. Pharmazeutische Zusammensetzung gemäß Anspruch 17 oder Anspruch 18, wobei die pharmazeutische Zusammensetzung für eine separate, sequenzielle oder gemeinsame Verabreichung in wirksamen Mengen, zusammen mit einer Trockenpulver-Inhalationsvorrichtung geeignet ist.

20. Pharmazeutische Zusammensetzung gemäß Anspruch 17, wobei die pharmazeutische Zusammensetzung für eine separate, sequenzielle oder gemeinsame Verabreichung in wirksamen Mengen, zusammen mit einer Inhalationsvorrichtung geeignet ist, **dadurch gekennzeichnet, dass** die Vorrichtung mindestens einen Verriegelungsmechanismus umfasst, der der Vorrichtung ermöglicht, in beiden Positionen, in denen die Vorrichtung für eine Inhalation einsatzbereit ist und sich die Klappe in der geschlossenen Position befindet, verriegelt zu bleiben, und des Weiteren der Vorrichtung ermöglicht, automatisch einen Set-up durchzuführen, wenn die Klappe geschlossen ist.

21. Pharmazeutisches Kit, umfassend die LAMAs und einen oder mehrere zusätzliche Wirkstoffe gemäß Definition in Anspruch 11 in separaten Dosierungeinheitsformen, wobei die Formen für eine separate, sequenzielle oder gemeinsame Verabreichung in wirksamen Mengen, zusammen mit einer oder mehreren Inhalationsvorrichtung(en) zur Verabreichung von LAMAs und einem oder mehreren zusätzlichen Wirkstoffen geeignet sind.

## Revendications

1. Composition pharmaceutique pour inhalation comprenant séparément ou conjointement des antagonistes muscariniques à action (LAMA) sous la forme d'une poudre sèche prolongée mélangés avec un excipient pharmaceutiquement acceptable qui comprend un mélange de particules fines ayant une taille moyenne de particule (d50) de 1,0 à 10,0 µm et des particules grossières ayant une taille moyenne de particule (d50) de 10,0 à 100,0 µm, la quantité de particules fines étant comprise entre 20 et 30 % en poids de la quantité totale de l'excipient.

2. Composition pharmaceutique selon la revendication 1, dans laquelle le rapport en poids des particules fines aux particules grossières est de 0,25 à 0,35 en poids.

3. Composition pharmaceutique selon la revendication 1, dans laquelle les particules fines dudit excipient pharmaceutiquement acceptable ont une taille moyenne de particule (d50) de 1,0 à 7,0 µm et les particules grossières ont une taille moyenne de particule (d50) de 10,0 à 75,0 µm.

4. Composition pharmaceutique selon l'une quelconque des revendications précédentes, dans laquelle l'excipient pharmaceutiquement acceptable est choisi dans le groupe constitué par le lactose, le mannitol, le mannitol séché par pulvérisation, le glucose, l'arabinose, le tréhalose, le cellobiose, le sorbitol, le maltitol, le xylitol, le saccharose, le maltose, le dextrane ou une combinaison de deux ou plus de ceux-ci.

5. Composition pharmaceutique selon la revendication 4, dans laquelle l'excipient pharmaceutiquement acceptable est de préférence le lactose ou le glucose ou le mannitol ou le mannitol séché par pulvérisation ou des mélanges de ceux-ci.

6. Composition pharmaceutique selon la revendication 4, dans laquelle l'excipient pharmaceutiquement acceptable est de préférence un mélange de lactose et de mannitol ou un mélange de lactose et de glucose ou un mélange de mannitol et de glucose.

7. Composition pharmaceutique selon la revendication 1 et l'une quelconque des revendications précédentes, dans laquelle la taille moyenne de particule (d₅₀) des LAMA est comprise entre 0,10 et 5,0 µm.

8. Composition pharmaceutique selon la revendication 1 et l'une quelconque des revendications précédentes, dans laquelle les LAMA sont choisis dans le groupe constitué par le tiotropium, le glycopyrronium, l'ipratropium, l'aclidinium, l'oxitropium, le daratropium ou un sel ou un ester pharmaceutiquement acceptable de ceux-ci, ou sous une forme énantiomériquement pure ou sous la forme d'un mélange racémique ou d'une combinaison de deux ou plus de ceux-ci.

9. Composition pharmaceutique selon la revendication 8, dans laquelle le LAMA est le tiotropium ou un sel ou un ester pharmaceutiquement acceptable de celui-ci, ou se présente sous une forme énantiomériquement pure ou sous la forme d'un mélange racémique.

10. Composition pharmaceutique selon la revendication 9, dans laquelle le tiotropium est présent sous forme de chlorure, de bromure, d'iodure, de méthanesulfonate, de paratoluènesulfonate ou de sulfate de méthyle de ceux-ci, de préférence sous forme de bromure.

11. Composition pharmaceutique selon la revendication 1, comprenant en outre un ou plusieurs agents actifs supplémentaires choisis parmi les bêta-agonistes à action prolongée, les bêta-2 agonistes à action courte, les corticostéroïdes ou une combinaison de deux ou plus de ceux-ci.

12. Composition pharmaceutique selon la revendication 11, dans laquelle les bêta-agonistes à action prolongée sont choisis dans le groupe constitué par le salmétérol, le formotérol, l'arformotérol, l'indacatérol, l'olodatérol, le vilantérol, le carmotérol, le bambutérol ou un sel ou un ester pharmaceutiquement acceptable de ceux-ci, ou se présentent sous une forme énantiomériquement pure ou sous forme d'un mélange racémique ou d'une combinaison de deux ou plus de ceux-ci.

13. Composition pharmaceutique selon la revendication 11, dans laquelle les bêta-2 agonistes à action courte sont choisis dans le groupe constitué par le salbutamol, le lévosalbutamol, la terbutaline, le pirbutérol, le procatérol, le fénotérol, le bitolterol, la ritodrine, le métaprotérénol ou un sel ou un ester pharmaceutiquement acceptable de ceux-ci, ou se présentent sous une forme énantiomériquement pure ou sous la forme d'un mélange racémique ou d'une combinaison de deux ou plus de ceux-ci.

14. Composition pharmaceutique selon la revendication 11, dans laquelle les corticostéroïdes sont choisis dans le groupe constitué par la fluticasone, le ciclésonide, le budésonide, la mométasone, la béclométhasone, la triamcinolone, le flunisolide, la dexaméthasone ou un sel ou un ester pharmaceutiquement acceptable de ceux-ci, ou se présentent sous une forme énantiomériquement pure ou sous la forme d'un mélange racémique ou d'une combinaison de deux ou plus de ceux-ci.

15. Composition pharmaceutique selon l'une quelconque des revendications précédentes, dans laquelle la quantité thérapeutiquement efficace de ladite composition pharmaceutique est administrée une fois par jour ou deux fois par jour.

16. Composition pharmaceutique selon l'une quelconque des revendications précédentes, destinée à être utilisée pour le traitement d'un état respiratoire choisi parmi l'asthme et la maladie pulmonaire obstructive chronique et d'autres maladies obstructives des voies respiratoires.

17. Composition pharmaceutique selon l'une quelconque des revendications précédentes, dans laquelle ladite composition pharmaceutique est appropriée pour être administrée séparément, séquentiellement ou conjointement en quantités efficaces, conjointement avec une plaquette thermoformée scellée étanche à l'humidité et à haute barrière.

18. Composition pharmaceutique selon l'une quelconque des revendications précédentes, dans laquelle ladite composition pharmaceutique est appropriée pour être administrée séparément, séquentiellement ou conjointement en quantités efficaces, conjointement avec une capsule.

19. Composition pharmaceutique selon la revendication 17 ou 18, dans laquelle ladite composition pharmaceutique est appropriée pour être administrée séparément, séquentiellement ou conjointement en quantités efficaces, conjointement avec un dispositif d'inhalation de poudre sèche.

20. Composition pharmaceutique selon la revendication 17, dans laquelle ladite composition pharmaceutique est appropriée pour être administrée séparément, séquentiellement ou conjointement en quantités efficaces, conjointement avec un dispositif d'inhalation, **caractérisée en ce que** ledit dispositif comprend au moins un mécanisme de verrouillage, permettant au dispositif de rester verrouillé dans les deux positions dans lesquelles le dispositif est prêt pour l'inhalation et le couvercle est en position fermée, et permettant en outre au dispositif de se réinstaller automatiquement lorsque le couvercle est fermé.

21. Trousse pharmaceutique comprenant les LAMA et un ou plusieurs agents actifs supplémentaires tels que définis dans la revendication 11, sous des formes posologiques unitaires distinctes, lesdites formes pouvant être administrées séparément, séquentiellement ou conjointement en quantités efficaces, conjointement avec un ou plusieurs dispositifs d'inhalation pour l'administration de LAMA et d'un ou de plusieurs agents actifs supplémentaires.
